# EUROPEAN PATENT SPECIFICATION

(11) **EP 2 208 471 B1**
(45) Date of publication and mention of the grant of the patent: **12.10.2011**
(21) Application number: 10004396.7
(22) Date of filing: 25.04.2006
(51) Int. Cl.: A61B 17/17, A61B 17/88, A61B 17/00, A61B 17/80

(54) **Medical system and system parts for spatially adjusting an aiming device relative to a body implant**
Medizinisches System und dazugehörige Systemteile zum räumlichen Einstellen eines Zielgerätes an einem Implantat
Système médical pour ajuster un guide de positionnement par rapport à un implant et éléments de ce système

(30) Priority: 17.01.2006 US 759376 P
(43) Date of publication of application: 21.07.2010
(62) Divisional of application: 06008537.0
(73) Proprietor: Stryker Trauma GmbH, 24232 Schönkirchen/Kiel (DE)
(72) Inventor: Prien, Ole, 24145 Kiel (DE); Cremer, Axel, 3428 Wiler (CH); Giersch, Helge, 24118 Kiel (DE)
(74) Representative: Maiwald Patentanwalts GmbH

(56) References cited:
- US-A- 5 676 666
- US-A1- 2004 015 174
- US-A1- 2004 204 717
- US-A1- 2005 085 818
- US-B2- 6 989 012

## Description

### FIELD OF INVENTION

The present invention relates to the field of surgery instruments. In particular, the present invention relates to a medical system for detachably establishing a spatial orientation between a body implant and an aiming device. More particular, the present invention relates to a medical system and parts of the system, which allow for a precise establishing of a relative spatial orientation between a bone stabilization implant and an aiming device for anchoring the implant to an internal part of a body.

Useful for understanding the invention, a method for detachably establishing a spatial orientation between a body implant and an aiming device, in particular between a bone stabilization implant and an aiming device for anchoring the implant to an internal part of a body is described.

### BACKGROUND OF THE INVENTION

In order to allow for a reliable stabilization of a broken bone in its normal position, special bone stabilizing implants are frequently used. Such implants are for instance metal plates, which are made e.g. from surgery steel. Such plates are usually fixed to the bone parts by means of threaded screws, which are driven into the bone tissue after so-called pre-drilled or pilot-drilled holes have been generated in the bone tissue. These pre-drilled holes allow for a reliable screwing procedure whereby the risk of further destroying the bone with the screw is significantly reduced.

In order to facilitate the drilling of these pre-drilled holes there are known so-called aiming devices, which work like a drilling jig. In this context an aiming device is detachably fixed to the metal plate in a spatial precise position.

Aiming devices are also used in connection with locking nails, which are driven into intra medullary tissue. Thereby, the position of a cross bore within the interlocking nail can be determined precisely. The cross bore is adapted to accommodate a fixing screw, which is driven crosswise through the corresponding bone section.

US 6,224,601 B1 discloses the use of an aiming device in an osteosynthesis auxiliary for the treatment of subtrochanteric, peritochanteric and femoral-neck fractures.

US 2004/0015174 A1 on which the preambles of claims 1 and 5 are based, discloses a surgical instrument guide assembly being positionable on a bone plate. The instrument guide assembly includes at least one guide member alignable with a corresponding fastener opening in the bone plate and a handle extending proximally from the guide member. A base is adapted to support the guide member on the bone plate. The base has a foot member engageable with the bone plate and a biasing member contactable with the bone plate.

There may be a need for a medical system and medical system parts for spatially adjusting an aiming device relative to a body implant, which allow for an easy, for a quick and for a reliable relative position adjustment.

### SUMMARY OF THE INVENTION

In order to fulfill the need defined above, an adaptor device, a positioning arm, and a medical system according to the independent claims are provided.

According to an aspect of the invention there is provided an adaptor device for detachably establishing a spatial orientation between a body implant and an aiming device, in particular for detachably establishing a spatial orientation between a bone stabilization implant and an aiming device for anchoring the implant to an internal part of a body. The adaptor device comprises an attachment element, which is designed such that an engaging element of a positioning arm can be hooked into the adaptor device. The adaptor device further comprises two interlocking elements which is designed such that a snapping mechanism of the positioning arm automatically engages in the interlocking elements when the positioning arm, when hooked into the adaptor device, is rotated relative to the adaptor device towards a final angular position and approaches the final position.

This aspect of the present invention is based on the idea that both a form-fit and a force-fit connection between the adaptor device and the positioning can be realized if the positioning arm is swiveled around a predefined center of rotation toward a final angular position.

The form-fit connection may be provided by the contour of the adaptor device, which contour complementarily fits to a contour being provided at the positioning arm. When the snapping mechanism is engaged in the interlocking elements, the contour of the adaptor device abuts against the contour of the positioning arm such that a form-fit connection is realized.

The force-fit connection may be provided if the adaptor device comprises a slightly different bending than the corresponding portion of the positioning arm, which portion abuts against the adaptor device when the snapping mechanism is engaged in the interlocking elements. Thereby, the adaptor device and/or the positioning arm are made from a material, which is at least partially resilient. When the form-fit connection between these two parts is established, at least one of these two parts is elastically deformed whereby the deformation force provides for the force-fit connection. Such a force-fit connection has the advantage that there is practically no spatial tolerance between the adaptor device and the positioning arm. Therefore, an in particular very precise spatial positioning between the adaptor device and the positioning arm may be established.

The snapping mechanism of the positioning arm automatically engages in the interlocking elements when the positioning arm has approached the final position. In the course of the whole movement the adaptor device is kept hooked into the adaptor device. The rotation is carried out around a rotation axis, which runs through a region wherein the attachment element and the engaging element abut against each other.

The adaptor device is adapted to be connected to a bone stabilizing implant. The adaptor device and the implant may be made from a single piece or from two or more individual pieces. However, it has to be ensured that the adaptor device and the implant may be rigidly fixed to each other.

The positioning arm is adapted to be connected to an aiming device. The positioning arm and the aiming device may be made of a single piece or of two or more individual pieces. In the latter case it has also to be ensured that the positioning arm and the aiming device are rigidly fixed to each other, such that the friction and/or form locked positioning arm provides a mechanically stable platform for the aiming device.

According to an embodiment of the present invention the attachment element is a projection salient from a body of the adaptor device. This may provide the advantage that the adaptor device can be manufactured easily out of a single piece of material.

It has to be noted that the attachment element may be any element, which allows that the positioning arm may be hooked into the adaptor device. For instance the attachment element may also be a recess, into which a projecting engaging element of the positioning arm engages.

According to a further embodiment of the invention the interlocking elements comprise an edge. The edge may be resilient such that a rigid or stiff element of the snapping mechanism can engage. Alternatively, the edge may by rigid such that a resilient and/or flexible element of the snapping mechanism can engage. A resilient and/or flexible element may be realized with a flexible material or by means of a spring type element, which prestresses the resilient element.

According to an example useful for understanding the invention the adaptor device comprises a further attachment element, which is designed in such a manner that a further engaging element of the positioning arm can be hooked into the further attachment element. This may have the advantage that a mechanically stable three-point bearing is automatically generated when the snapping mechanism of the positioning arm engages in the interlocking element.

Furthermore, when two attachment elements are involved in order to provide a mechanical coupling between the adaptor device and the positioning arm, the rotation axis of the positioning arm is defined precisely by the spatially arrangement of two regions wherein the two attachment elements and the two engaging elements abut against each other, respectively. Therefore, an operator and in particular a surgeon can easily and effectively attach the positioning arm to the adaptor device in a reliable spatial position.

It is pointed out that a stable three-point bearing is realized in accordance with the invention, by means of only one attachment element but two interlocking elements, the interlocking element described above and a further interlocking element. Thereby, the rotation of the positioning arm is carried out with respect to a center of rotation or a pivotal point such the rotation axis is spatially not exactly defined. However, an operator or a surgeon will have no problems to manually control the rotation such that the snapping mechanism will engage in the interlocking element.

According to the above example the further attachment element is a further projection salient from a body of the adaptor device. As has been described above with regard to the attachment element, also the further attachment element may be a recess, into which a further projecting engaging element engages.

According to a further embodiment of the invention the adaptor device is formed integrally with the implant. This may provide the advantage, that there is no additional effort necessary in order to provide a rigid and mechanically reliable connection between the adaptor device and the implant.

However, it has to be pointed out that as an alternative the adaptor element may also be formed separately from the implant. In this case, one has to ensure that a rigid and mechanically reliable connection between the adaptor device and the implant is provided. This may be done e.g. be using appropriate coupling elements like screws, pins, nails, rivets, et cetera.

According to a further embodiment of the invention the adaptor device is formed integrally with an internal fixator and in particular with a plate, which is used for bone stabilization. Such an internal fixator or such a plate may be used in order to fix a broken bone in its anatomical correct position. Thereby, the plate is attached to different parts of the bone by means of screws, pins, loops, et cetera. In particular when screws are used for attaching the plate, an aiming device may be very helpful, which aiming device comprises a drilling jig. Thereby, an operator and in particular a surgeon is capable of precisely drilling holes in the bone, whereby each hole is formed exactly at the proper position with respect to a corresponding through hole in the plate.

According to a further aspect of the invention there is provided a positioning arm for detachably establishing a spatial orientation between a body implant and an aiming device, in particular for detachably establishing a spatial orientation between a bone stabilization implant and an aiming device for anchoring the implant to an internal part of a body. The positioning arm comprises an engaging element, which is designed in such a manner that it may be coupled to an attachment element of an adaptor device such that the positioning arm is hooked into the adaptor device. The positioning arm further comprises a snapping mechanism, which is designed to automatically engage in an interlocking element of the adaptor device, when the positioning arm, when hooked into the adaptor device, is rotated relative to the adaptor device towards a final angular position.

According to an embodiment of the invention the engaging element is a notch. The notch may be formed directly in the body of the positioning arm. Preferably, the notch is formed in a projection extending from the body of the positioning arm.

According to a further embodiment of the invention the snapping mechanism comprises a hook type element, which is adapted to engage in the interlocking element. Preferably, the hook type element is resilient such that it is capable to engage in a stiff interlocking element. However, it should be clear that the positioning arm might also be realized with a stiff or inflexible hook type element, which is adapted to engage in a resilient interlocking element or at least in resilient part of the interlocking element.

A resilient hook type element may be realized with a flexible material or by means of a spring element, which prestresses the hook type element.

According to another embodiment useful for understanding the invention the positioning arm comprises a further engaging element, which is designed in such a manner that it may be coupled to a further attachment element of the adaptor device. As has already been mentioned above, the use of a further engaging element in connection with a corresponding further attachment element provides the advantage that the rotation axis of the positioning arm is defined precisely. Furthermore, a mechanically stable three-point bearing is automatically generated when the snapping mechanism of the positioning arm engages in the interlocking element.

In this context it is again noted that in accordance with the invention, a stable three-point bearing is realized with only one attachment element but with two interlocking elements. Thereby, the rotation of the positioning arm is carried out with respect to a center of rotation or a pivotal point. In that case, the orientation of the rotational axis is defined by a single reference point only. This means, that an operator and in particular a surgeon has to take care that the positioning arm is moved properly such that, when reaching the final angular position of the positioning arm, the interlocking element and the snapping mechanism provide a mechanically reliable connection between the adaptor device and the positioning arm.

According to the above embodiment, the further engaging element is a further notch. As has been described above with respect to the attachment element also the further attachment element may be a recess into which a further projecting engaging element engages.

The further notch may be formed in the same manner; in a similar manner or in a different manner with respect to the notch described above. Therefore, the further notch can be formed directly in the body of the positioning arm. Preferably, the further notch is formed in a projection extending from the body of the positioning arm.

According to a further embodiment of the invention the positioning arm further comprises a handle for facilitating a manual rotation of the positioning arm with respect to the adaptor device. Preferably, the handle is formed in an ergonomic manner. This has the advantage that an easy use of the positioning arm is guaranteed even if the surgeon is under a physical and/or under a mental stress condition, which frequently occurs in particular during complicated and elaborate surgeries.

According to a further embodiment of the invention the positioning arm further comprises a locking device, which is adapted to lock the snapping mechanism. The locking device has the advantage that an unintentional opening of the mechanical connection between the snapping mechanism and the interlocking element may be prevented effectively. Of course, the positioning arm may be removed from the adaptor device by an appropriate movement of the locking device after surgery procedure using the aiming device has been successfully carried out.

According to a further embodiment of the invention the locking device comprises a rotatable lever. Preferably, the rotatable lever is integrated in a handle such that the locking device can be realized without requiring additional space.

According to a further embodiment of the invention the positioning arm is formed integrally with the aiming device. This may provide the advantage, that there is no additional effort necessary in order to provide a rigid and mechanically reliable connection between the positioning arm and the aiming device. Furthermore, an accidentally wrong assembly of the aiming device with the positioning arm may be prevented effectively.

According to a further embodiment of the invention the positioning arm is adapted to be detachably connected to the aiming device. This may provide the advantage that the aiming device can be formed separately from the positioning arm. It is clear that in this case one has to ensure that a rigid and mechanically reliable connection between the positioning arm and the aiming device is provided.

A configuration wherein the positioning arm and the aiming device are formed as separate objects may provide the advantage that the implant, the adaptor device, the positioning arm and the aiming device can be included in a modular construction kit wherein each of these items is included in a variety of slightly different dimensions. Therefore, depending on the respective requirements the best combination of these items can be selected such that an optimal surgery may be accomplished.

According to a further aspect of the invention there is provided a medical system for detachably establishing a spatial orientation between a body implant and an aiming device, in particular between a bone stabilization implant and an aiming device for anchoring the implant to an internal part of a body. The medical system comprises an adapter device as defined in claims 1 to 5. The medical system further comprises a positioning arm as defined in claims 6 to 13.

Useful for understanding the invention there is described a method for detachably establishing a spatial orientation between a body implant and an aiming device, in particular between a bone stabilization implant and an aiming device for anchoring the implant to an internal part of a body. The method comprises the steps of
(a) bringing together a positioning arm and an adaptor device in such a manner that an engagement element of the positioning arm is coupled to an attachment element such that the positioning arm is hooked into the adaptor device,
(b) rotating the positioning arm relative to the adaptor device towards a final angular position, and
(c) intervening a snapping mechanism of the positioning arm in an interlocking element of the adaptor device.

The invention is based on the idea that a surgeon may establish a stable and reliably mechanical contact between the positioning arm and the adaptor device by means of a simple movement of his hand. Thereby, it is only necessary to perform a small rotational or swiveling movement of the positioning arm with respect to the adaptor device in order to allow for a mechanical snapping in connection between the positioning arm and the adaptor device.

The step of intervening may occur automatically when the positioning arm approaches the final angular position. This makes it in particular easy for a surgeon to establish the stable and reliably mechanical contact between the positioning arm and the adaptor device such that it is not necessary to perform an extra movement of the hand in order to provide for a firm mechanical connection between the positioning arm and the adaptor device.

The method may further comprise the step of locking the snapping mechanism by means of a locking device. This embodiment has the advantage that an unintentional opening of the mechanical connection between the snapping mechanism and the interlocking element may be prevented in an easy and effective way.

After a medical procedure employing the aiming device has been accomplished the positioning arm can easily be removed from the adaptor device and/or from the implant by simply removing or by simply opening the locking device. Such a medical procedure can comprise for instance boring holes in a bone with the help of a drilling jig being provided by the aiming device.

The step of locking the snapping mechanism may comprise rotating a lever. The rotatable lever may be integrated in a handle such that the locking device can be realized without requiring additional space. Therefore, the positioning arm does not comprise sharp edges and corners, which could bear the risk that internal tissue of a patient is damaged.

It has to be noted that embodiments of the invention have been described with reference to different subject matters. In particular, some embodiments have been described with reference to different apparatus type claims. However, a person skilled in the art will gather from the above and the following description that, unless other notified, in addition to any combination of features belonging to one subject matter also any combination between features relating to different subject matters, is disclosed with this application.

The aspects defined above and further aspects of the present invention may be understood from the examples of embodiment to be described hereinafter and may be explained with reference to the examples of embodiment. The invention will be described in more detail hereinafter with reference to examples of embodiment although these examples do not show all features of the invention.

### Brief Description of the Drawings

Figures 1a to 1e show different views of a bone stabilizing plate including an integrally formed adaptor device.
Figures 2a to 2d show different views of a bone stabilizing medical system according to an exemplary embodiment.
Figures 3a to 3n illustrate the procedure for establishing a firm mechanical connection between the positioning arm and the adaptor device.

### Detailed Description

The illustration in the drawing is schematically. It is noted that in different drawings, similar or identical elements are provided with the same reference signs or with reference signs, which are different from the corresponding reference signs only within the first digit.

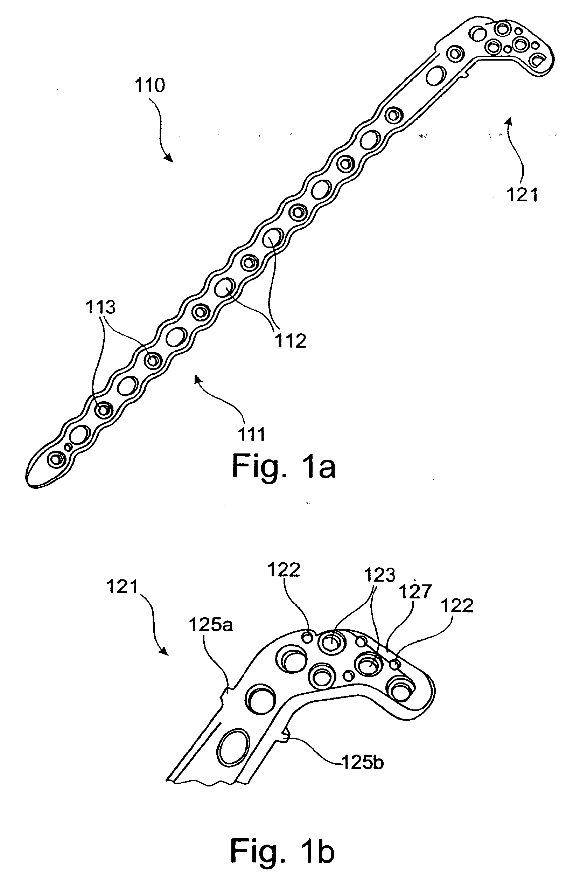

As can be seen from figure 1a, a bone stabilizing plate 110 comprises a lower portion 111 and an upper portion 121. The upper portion represents an adaptor device121, which is designed in such a manner that a firm mechanical connection between the plate 110 and a positioning arm may be established very easily.

The bone stabilizing plate comprises a plurality of openings 112 and a plurality of through holes 113. The openings are formed in the plate 111 in order to reduce the weight of the plate 111. The through holes 113 may be used for inserting screws (not depicted) for fixing the plate 111 to a bone or to a bone fragment such that all parts of the bone are attached to the bone stabilizing plate 111.

Figure 1b shows an enlarged view of the adaptor device 111. The adaptor device 111 comprises also some openings 122 and some through holes 123, which may be uses for the same purposes as the openings 112 and as the plurality of through holes 113 shown in figure 1a, respectively.

The upper part 121 of the plate 111 is bent such that the plate 111 may be abutted against a broken bone along the full length of the bone including the bone end, e.g. the proximal tibia and the distal femur.

The adaptor device 121 comprises two attachment elements, a first attachment element 125a and a second attachment element 125b. The two attachment elements are formed as projections salient from a body 126 of the adaptor device 111. The adaptor device 121 further comprises an interlocking element127 having the shape of an edge. As will be described later on, the attachment elements 125a and 125b and the interlocking element 127 may provide both a stable three-point bearing and a secure detachable connection between a positioning arm and the plate 111.

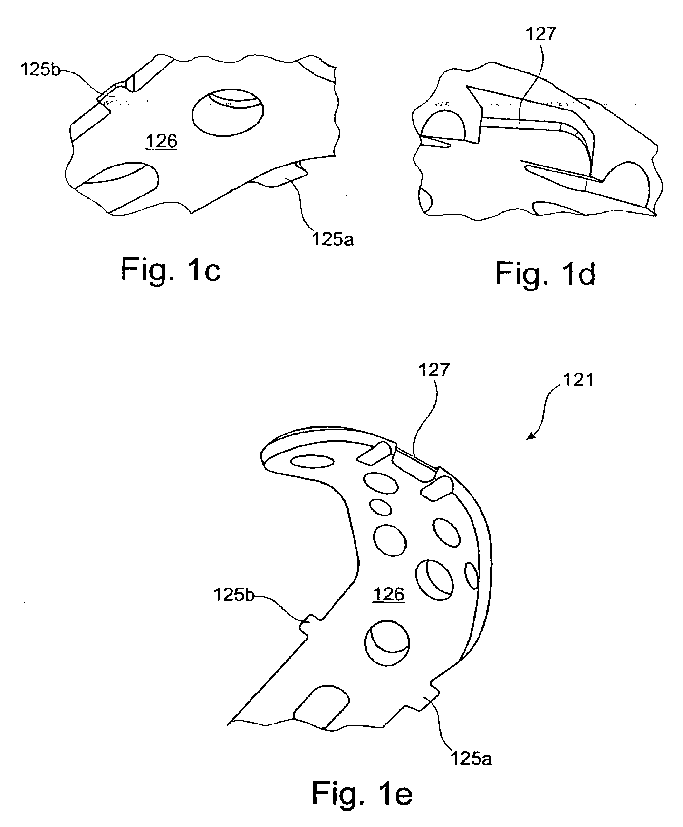

Figure 1c show the two attachment element 125a and 125b sticking out from the body 126 of the adaptor device 111.

Figure 1d shows the interlocking element 127, which according to the embodiment shown here is formed at the almost most upper section of the plate 110. The edge of the interlocking element 127 can be described by a recess which is open to the bone contacting surface of the plate 110 and which extends inwardly from the plate edge and upwardly towards the top surface of the plate.

Figure 1e shows a further view of the adaptor device 121. The two attachment elements 125a and 125b are formed as projections protruding from the body 126. The interlocking element 127 is formed as a more or less sharp edge at the outer circumference of the adaptor device 111.

The adaptor device 121 comprises an asymmetric shape wherein the interlocking element 127 is formed slightly off a longitudinal axis of the bone stabilizing plate 111.

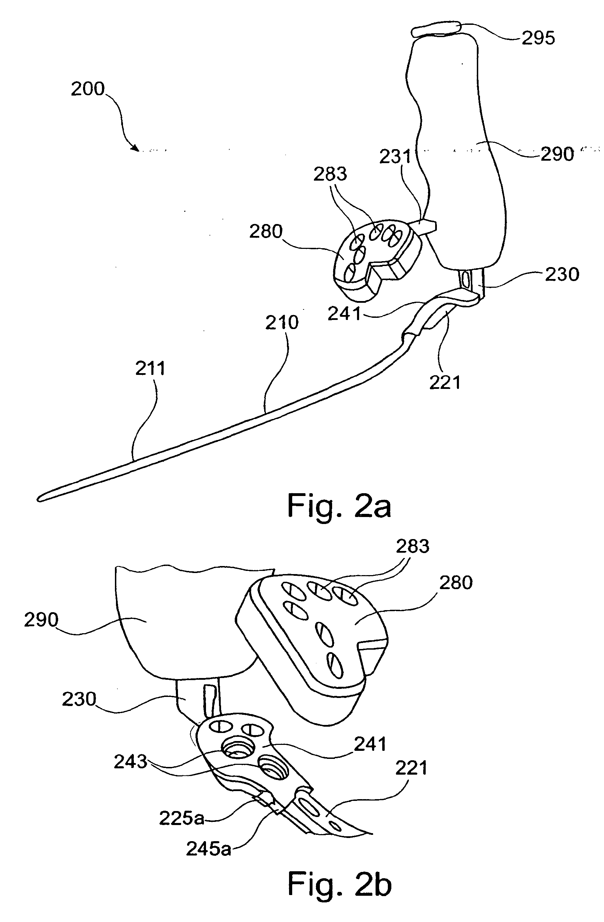

Figure 2a shows a general view of a medical system 200. The medical system 200 allows for a convenient care of a broken bone by means of the bone stabilizing plate 210, which comprises a lower portion 211 and an upper portion 221. The upper portion 221 represents an adaptor device, which allows a positioning arm 230 to be detachably but firmly connected to the plate 210.

The positioning arm 230 comprises a lower portion 241 and an upper portion 231. The lower portion represents a contact portion 241, which is adapted to be detachably and firmly connected to the adaptor device 221.

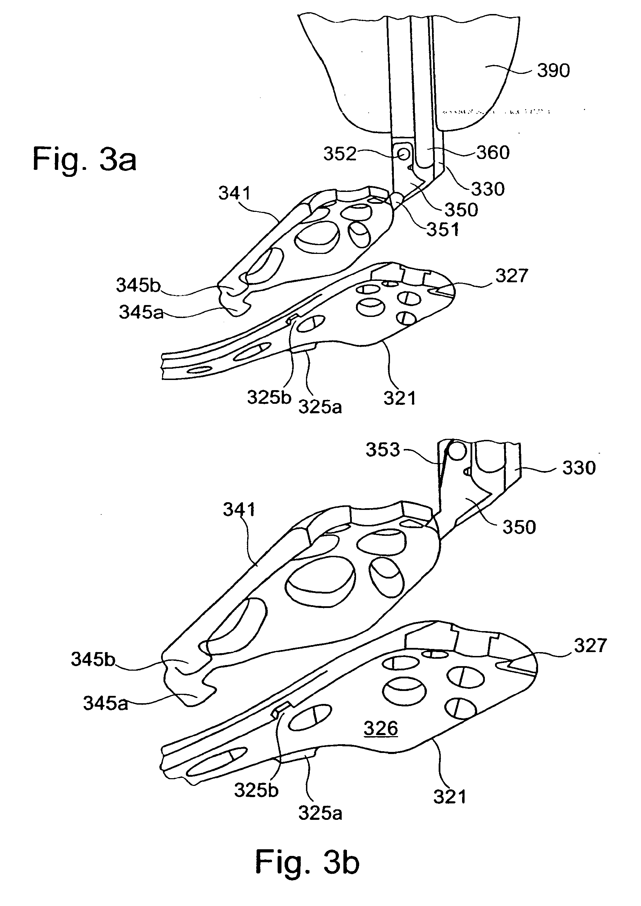

The positioning arm 230 further comprises a handle 290. The handle 290 is formed in an ergonomic manner such that the positioning arm 230 may be moved manually in an easy and convenient manner. The handle is equipped with a rotatable lever 295, which is mechanically coupled to a locking device. The locking device will be described later on in connection with the figures 3a to 3g.

The upper portion 231 of the positioning arm 230 represents a holder for an aiming device 280. The aiming device 280 represents a drilling jig comprising through holes 283 for a precise and an easy pre-drilling of holes into a bone fragment, which is located directly beneath the upper portion 221 of the bone stabilizing plate 210. Thereby, a minimal invasive and percutaneous surgery technique may be realized.

Figure 2b shows an enlarged view of the connection portion between the positioning arm 230 and the adaptor device 221. The contact portion 241 of the positioning arm 230 comprises holes 243. These holes 243 represent openings such that a drill tool may be passed through the contact portion 241 in order to pre-drill holes in a bone fragment located directly beneath the adaptor device 221. The holes 243 have a diameter, which is bigger than the diameter of the through holes 123 in the adapter device 121 (see figure 1b). This makes it possible that screws, which are used for fixing the adaptor device 221 to the bone section located beneath the adaptor device 221, may be passed through the holes 243 formed in the contact portion 241.

At the lower end of the contact portion 241 there are provided two engaging elements, a first engaging element 245a and a second engaging element, respectively. The second engaging element cannot be seen in the view shown in figure 2b. According to the embodiment described here the engaging elements are formed as two notches, a first notch 245a and a second notch, respectively. The two notches have such a shape that the contact portion 241 of the positioning arm 230 may be hooked into the two attachment elements, the first attachment element 225a and the second attachment element, respectively.

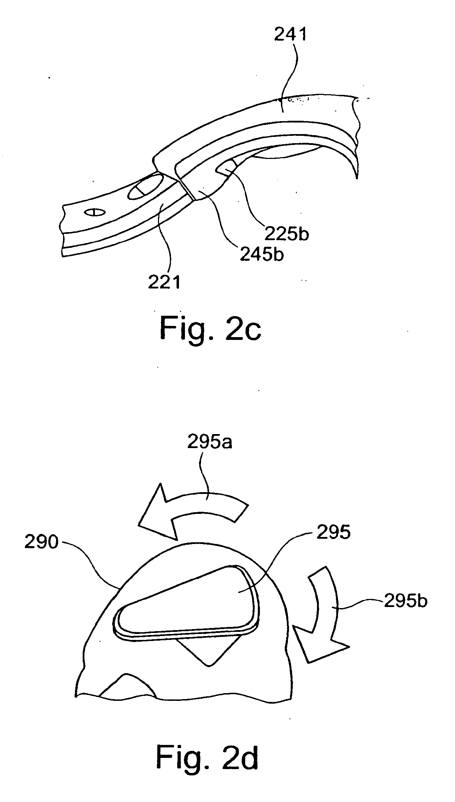

Figure 2c shows a further enlarged view of the connection portion between the contact portion 241 of the positioning arm 230 and the adaptor device 221. In this view the second engaging element (second notch) 245b can be seen as it hooks into the second attachment element (second projection) 225b.

Figure 2d shows a top view of the handle 290. On top of the handle 290 there is provided a small lever 295. The lever 295 can be rotated in a clockwise direction 295b for interlocking the detachable connection between the adaptor device 221 of the bone stabilizing plate 210 and the positioning arm 230 representing a mechanical holder for the aiming device 280. The lever 295 can also be rotated in a anti clockwise direction 295a in order to release the interlocking device such that the positioning arm 230 can be removed from the bone stabilizing plate 210.

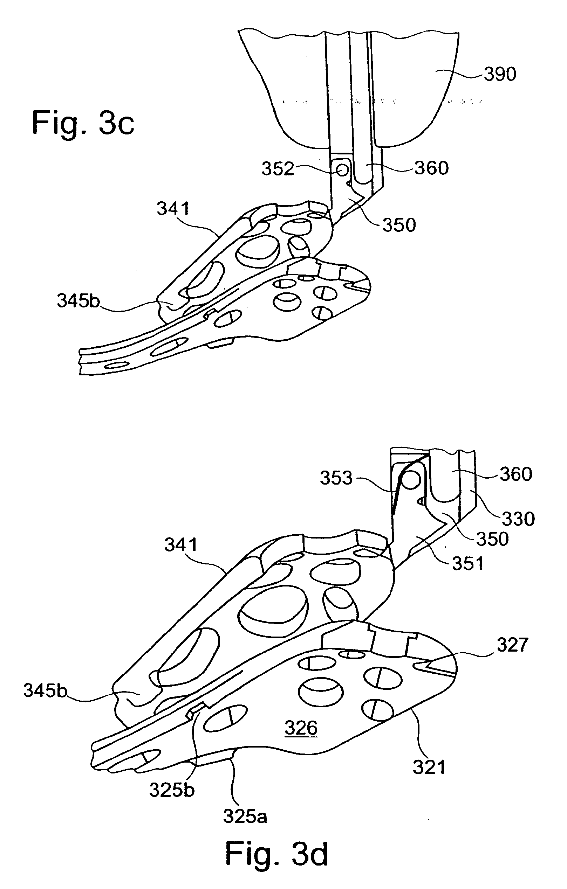

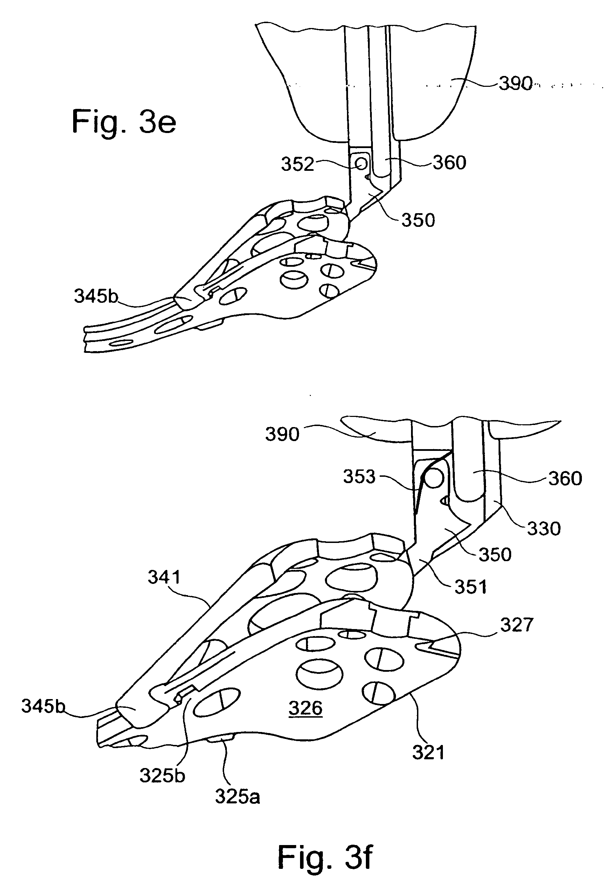

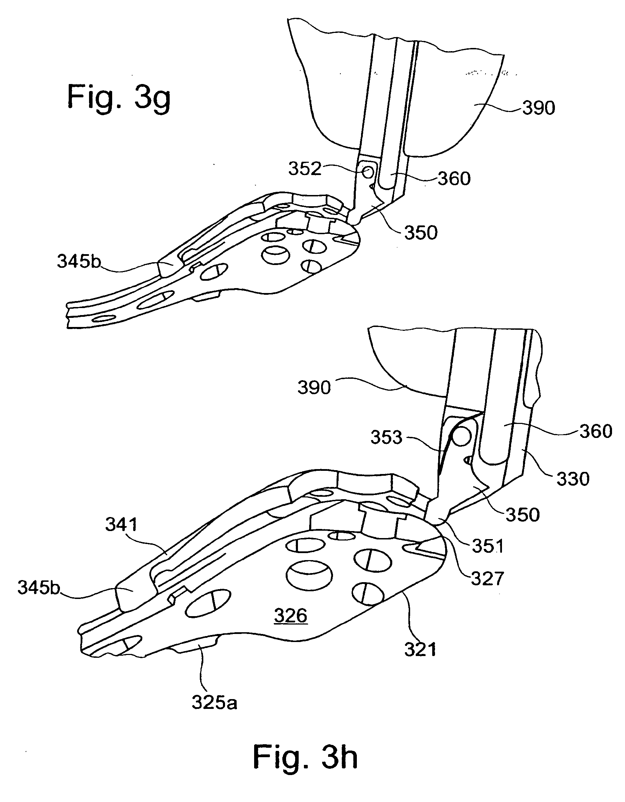

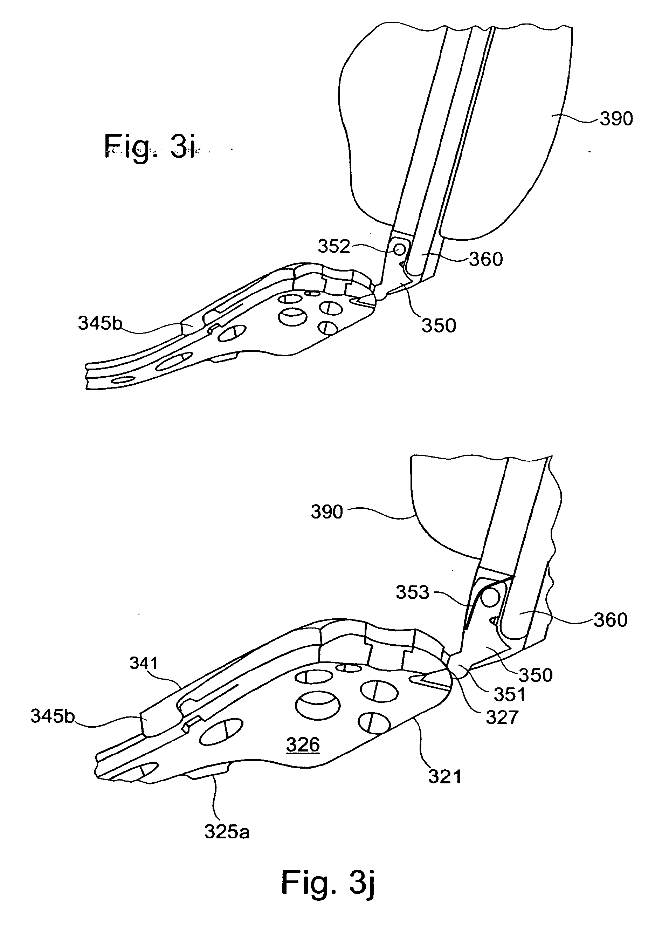

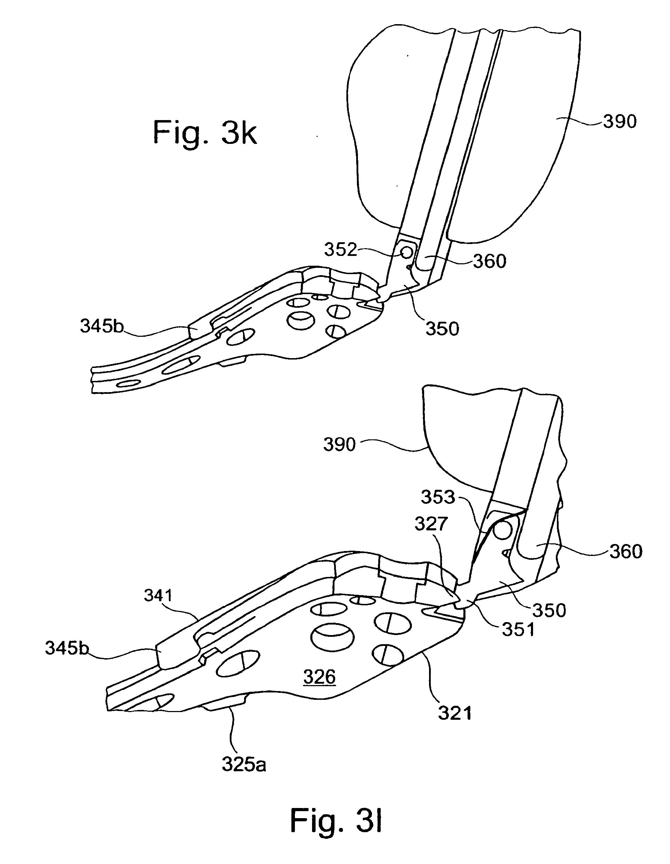

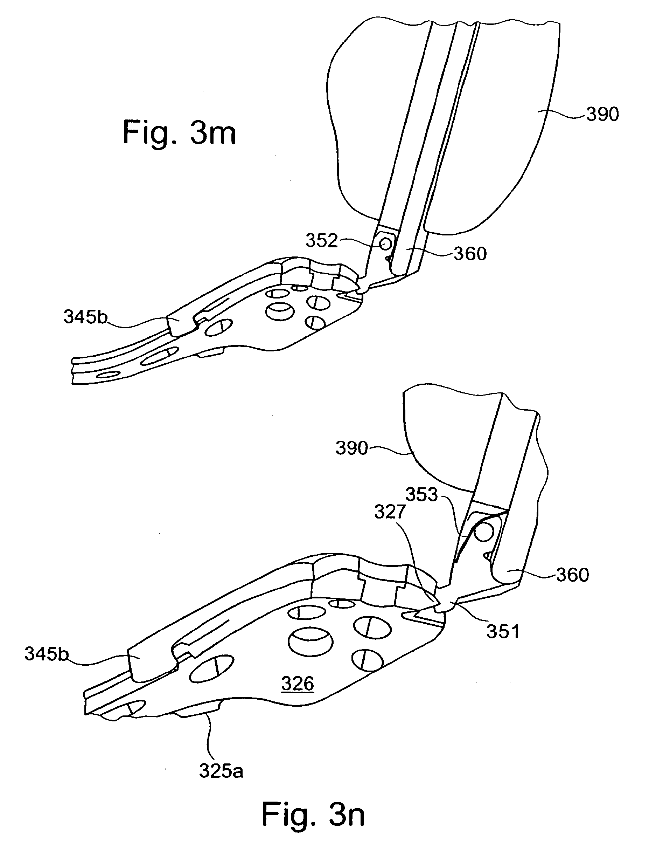

Figures 3a to 3n show a sequence of views illustrating the procedure of establishing a firm mechanical connection between the contact portion 341 of the positioning arm 330 and the adaptor device 321. The Figures 3a, 3c, 3e, 3g, 3i, 3k and 3m show a general view of the relative orientation between the contact portion 341 and the adaptor device 321. The Figures 3b, 3d, 3f, 3h, 3j, 3l and 3n each show a corresponding enlarged view.

As can be seen from Figures 3a and 3b, an operator (not shown) moves the contact portion 341 towards the adaptor device 321 in an appropriate orientation such that the first notch 345a and the second notch 345b hooks into the first projection 325a and the second projection 325b, respectively. The two projections 325a and 325b stick out from the body 326 of the adaptor device 321.

The positioning arm 330 is provided with a snapping mechanism 350, which is rotatable with respect to a pivot 352 within small angular limits. The snapping mechanism 350 is provided with a spring 353 such that the snapping mechanism 350 is kept in the initial angular position shown in figure 3a.

The snapping mechanism 350 comprises a hook type element 351, which is integrally formed with the snapping mechanism 350. The function of the snapping mechanism 350 will be described later on.

The positioning arm 330 is further provided with a locking device 360. According to the embodiment described herein the locking device is a rod 360, which is mechanically coupled to the lever 295 shown in Figure 2d. As can be easily seen, the locking rod 360 is capable of seizing the snapping mechanism 350, when the locking rod 360 is moved downwards such that it touches the snapping mechanism 350. The locking rod 360 is mechanically coupled to the lever 295 such that, when the lever 295 is rotated clockwise, the locking rod 360 moves downward. When the lever 295 is rotated counter clockwise the locking rod 360 moves upward. The man skilled in the art will find various appropriate solutions in order to realize the described mechanical coupling between the lever 295 and the locking rod 360.

The adaptor device 321 is provided with the interlocking element 327 having the shape of an edge. As can also easily been seen from Figure 3a, the snapping mechanism 350 is adapted to engage in the interlocking element 327, when the contact portion 341 and the adaptor element 321 are abutted against each other.

In order to establish a detachable connection between the contact portion 341 and the adaptor element 321, the positioning arm 330 is manually moved towards the adaptor element 321. This is depicted in Figures 3c and 3d.

As can be seen from Figures 3e and 3f, the contact portion 341 is further moved towards the adaptor element 321, such that the two notches 345a and 345b hook into the two projections 325a and 325b, respectively.

As can be seen from Figures 3g and 3h, the positioning arm is pivotally rotated around an axis which is defined by the two regions wherein the two attachment elements 325a and 325b and the two engaging elements 345a and 345b abut against each other, respectively. Thereby, the snapping mechanism 350 and the interlocking element 327 approach against each other. When the hook type element 351 of the snapping mechanism 350 abuts against an upper side of the interlocking element 327 the snapping mechanism 350 will be pulled to the side.

Figures 3i and 3j show the contact portion 341 and the adaptor element 321 when abutted against each other. However, the snapping mechanism 350 has not yet been moved back in its normal position.

Figures 3k and 3l show the contact portion 341 and the adaptor element 321, which are fastened to each other by means of the snapping mechanism 350. Due to an elastic force powered by the spring 353 the snapping mechanism 350 has been moved back in its normal position. Thus, the contact portion 341 and the adaptor element 321 are secured to each other by means of a three-point bearing. The three-point bearing includes the connection between the snapping mechanism 350 and the interlocking element 327 and the two regions wherein the two attachment elements and the two engaging elements abut against each other, respectively. Thereby, a precise orientation between the plate 210 and the aiming device 280 may be established (see Figure 2a).

Figures 3m and 3n show a locked state of the snapping mechanism 350. The locked state is characterized by the fact that an accidental and unwanted opening of the snapping mechanism 350 can be prevented reliably. Thereby, the locking rod 360 is moved downwards such that the lower end of the rod 360 engages in the snapping mechanism 350.

When the snapping mechanism 350 is in the locked state the bone stabilizing plate respectively the adaptor device 321 and the aiming device respectively the positioning arm 330 represent a stiff medical system. Therefore, a surgeon may grab the medical system at the handle 390 and position the plate on a broken bone of a patient. The aiming device allows for a precise pre-drilling of holes within the bone tissue, wherein the predrilled holes align with predetermined holes within the bone stabilizing plate. Thereby, the aiming device works like a drilling jig. After having completed the pre-drilling, threaded screws, driven into the bone tissue in order to stabilize the bone in its normal position.

After the plate has been fixed to the broken bone the positioning arm including the aiming device has to be removed from the fixed plate in order to allow for a closing of the operation trauma. The closing typically ends by sewing up the patient's skin.

In order to remove the positioning arm from the bone stabilizing plate one has to detach the contact portion 341 from the adaptor element 321. Thereby, first the locking rod 360 has to be moved upwards. Then, the positioning arm is rotated slightly counter clockwise such that the snapping mechanism 350 glides off the interlocking element 327. The detaching of the contact portion 341 from the adaptor element 321 may be completed by carrying out the above described procedure in a reversed order.

In the following, further exemplary embodiments useful for understanding the invention are described.

According to a first embodiment, an adaptor device for detachably establishing a spatial orientation between a body implant 210 and an aiming device 280, in particular between a bone stabilization implant 210 and an aiming device 280 for anchoring the implant 210 to an internal part of a body, comprises an attachment element 225a, which is designed such that an engaging element 245a of a positioning arm 230 can be hooked into the adaptor device 221, and an interlocking element 327, which is designed such that a snapping mechanism 350 of the positioning arm 330 automatically engages in the interlocking element 327 when the positioning arm 330, when hooked into the adaptor device 321, is rotated relative to the adaptor device 321 towards a final angular position.

The attachment element 325a of the adaptor device may be a projection salient from a body 326 of the adaptor device 321.

The interlocking element 327 of the adaptor device may comprise an edge.

The adaptor device may further comprise a further attachment element 325b, which is designed such that a further engaging element 345b of the positioning arm 330 can be hooked into the further attachment element 325b, wherein the further attachment element 325b may be a further projection salient from a body 326 of the adaptor device 321.

The adaptor device may be formed integrally with the implant 210.

The adaptor device may be formed integrally with an internal fixator 210, in particular with a plate 210 which is used for a bone stabilization.

According to a second embodiment, a positioning arm for detachably establishing a spatial orientation between a body implant 210 and an aiming device 280, in particular between a bone stabilization implant 210 and an aiming device 280 for anchoring the implant 210 to an internal part of a body, comprises an engaging element 345a, which is designed to be coupled to an attachment element 325a of an adaptor device 321 such that the positioning arm 330 is hooked into the adaptor device 321, and a snapping mechanism 350, which is designed to automatically engage in an interlocking element 327 of the adaptor device 321, when the positioning arm 330, when hooked into the adaptor device 321, is rotated relative to the adaptor device 321 towards a final angular position.

The engaging element 345a of the positioning arm may be a notch.

The snapping mechanism 350 of the positioning arm may comprise a hook type element 351, which is adapted to engage in the interlocking element 327.

The positioning arm may further comprise a further engaging element 345b, which is designed to be coupled to a further attachment element 325b of the adaptor device 321. wherein the further engaging element 345b may be a further notch.

The positioning arm may further comprise a handle 290 for facilitating a manual rotation of the positioning arm 230 with respect to the adaptor device 221.

The positioning arm may further comprise a locking device 360, adapted to lock the snapping mechanism 350, wherein the locking device 360 may comprise a rotatable lever 295.

The positioning arm may be formed integrally with the aiming device 280.

The positioning arm may be adapted to be detachably connected to the aiming device 280.

According to a third embodiment, a system for detachably establishing a spatial orientation between a body implant 210 and an aiming device 280, in particular between a bone stabilization implant 210 and an aiming device 280 for anchoring the implant 210 to an internal part of a body, comprises an adapter device 221 according to the first embodiment, and a positioning arm 230 according to the second embodiment.

According to a fourth embodiment, a method for detachably establishing a spatial orientation between a body implant 210 and an aiming device 280, in particular between a bone stabilization implant 210 and an aiming device 280 for anchoring the implant 210 to an internal part of a body, comprises the steps of bringing together a positioning arm 230 and an adaptor device 221 in such a manner that an engagement element 345a of the positioning arm 330 is coupled to an attachment element 325a such that of the positioning arm 330 is hooked into the adaptor device 321, rotating the positioning arm 330 relative to the adaptor device 321 towards a final angular position, and intervening a snapping mechanism 350 of the positioning arm 330 in an interlocking element 327 of the adaptor device 321.

The step of intervening may occur automatically when the positioning arm 330 approaches the final angular position.

The method may further comprise the step of locking the snapping mechanism 350 by means of a locking device 360, wherein the step of locking the snapping mechanism 360 may comprise rotating a lever 295.

It should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

### List of reference signs:

- 110: plate
- 111: lower portion
- 112: openings
- 113: through holes
- 121: upper portion / adaptor device
- 122: opening
- 123: through holes
- 125a: first attachment element / first projection
- 125b: second attachment element / second projection
- 126: body of adaptor device 121
- 127: interlocking element / edge

- 200: medical system
- 210: plate
- 211: lower portion
- 221: upper portion / adaptor device
- 225a: first projection / first attachment element
- 225b: second projection / second attachment element
- 230: positioning arm
- 231: upper portion
- 241: lower portion / contact portion
- 243: holes in contact portion 241
- 245a: first engaging element / first notch
- 245b: second engaging element / second notch
- 280: aiming device
- 283: though holes / drilling jig
- 290: handle
- 295: rotatable lever
- 295a: opening direction
- 295b: closing direction

- 321: upper portion / adaptor device
- 325a: first attachment element / first projection
- 325b: second attachment element / second projection
- 326: body of adaptor device
- 327: interlocking element / edge
- 330: positioning arm
- 341: lower portion / contact portion
- 350: snapping mechanism
- 351: hook type element
- 352: pivot
- 353: spring
- 345a: first engaging element / first notch
- 345b: second engaging element / second notch
- 360: locking device / locking rod
- 390: handle

## Claims

1. An adaptor device adapted to be connected to a body implant (210) for detachably establishing a spatial orientation between said body implant (210) and an aiming device (280), in particular between a bone stabilization implant (210) and an aiming device (280) for anchoring the implant (210) to an internal part of a body, the adaptor device (221, 321) comprising:
one attachment element (225a, 325a), which is designed such that an engaging element (245a, 345a) of a positioning arm (230, 330) can be hooked into the adaptor device (221, 321), and
an interlocking element (327), which is designed such that a snapping mechanism (350) of the positioning arm (230, 330) automatically engages in the interlocking element (327) when the positioning arm (230, 330), when hooked into the adaptor device (221, 321), is rotated relative to the adaptor device (221, 321) towards a final angular position, **characterized in that** said adaptor device comprises a further interlocking element (327), which is also designed such that a snapping mechanism (350) of the positioning arm (230, 330) automatically engages in the interlocking element (327) when the positioning arm (230, 330), when hooked into the adaptor device (221, 321), is rotated relative to the adaptor device (221, 321) towards a final angular position.

2. The adaptor device according to claim 2, wherein
the attachment element (225a, 325a) is a projection salient from a body (326) of the adaptor device (221, 321).

3. The adaptor device according to any one of the claims 1 to 2, wherein
each of the interlocking elements (327) comprises an edge.

4. The adaptor device according to any one of the claims 1 to 3, wherein
the adaptor device (221, 321) is formed integrally with the implant (210).

5. The adaptor device according to any one of the claims 1 to 3, wherein
the adaptor device (221, 321) is formed integrally with an internal fixator (210), in particular with a plate (210) which is used for a bone stabilization.

6. A positioning arm for detachably establishing a spatial orientation between a body implant (210) and an aiming device (280), in particular between a bone stabilization implant (210) and an aiming device (280) for anchoring the implant (210) to an internal part of a body, the positioning arm (230, 330) comprising:
one engaging element (245a, 345a), which is designed to be coupled to an attachment element (225a, 325a) of an adaptor device (221, 321) adapted to be connected to said body implant (210) such that the positioning arm (230, 330) is hooked into the adaptor device (221, 321), and
a snapping mechanism (350), which is designed to automatically engage in an interlocking element (327) of the adaptor device (221, 321), when the positioning arm (230, 330), when hooked into the adaptor device (221, 321), is rotated relative to the adaptor device (221, 321) towards a final angular position, **characterized in that** said positioning arm (230, 330) comprises a further snapping mechanism (350), which is also designed to automatically engage in a further interlocking element (327) of the adaptor device (221, 321), when the positioning arm (230, 330), when hooked into the adaptor device (221, 321), is rotated relative to the adaptor device (221, 321) towards a final angular position.

7. The positioning arm according to claim 6, wherein
the engaging element (245a, 345a) is a notch.

8. The positioning arm according to any one of the claims 6 to 7, wherein
each of the snapping mechanisms (350) comprise a hook type element (351), which is adapted to engage in one of the interlocking elements (327).

9. The positioning arm according to any one of the claims 6 to 8, further comprising
a handle (290, 390) for facilitating a manual rotation of the positioning arm (230, 330) with respect to the adaptor device (221, 321).

10. The positioning arm according to any one of the claims 6 to 9, further comprising
a locking device (360), adapted to lock one of the snapping mechanisms (350).

11. The positioning arm according to claim 10, wherein
the locking device (360) comprises a rotatable lever (295).

12. The positioning arm according to any one of the claims 6 to 11, wherein
the positioning arm (230, 330) is formed integrally with the aiming device (280).

13. The positioning arm according to any one of the claims 6 to 11, wherein
the positioning arm (230, 330) is adapted to be detachably connected to the aiming device (280).

14. A system for detachably establishing a spatial orientation between a body implant (210) and an aiming device (280), in particular between a bone stabilization implant (210) and an aiming device (280) for anchoring the implant (210) to an internal part of a body, the system comprising:
an adapter device (221, 321) according to any one of the claims 1 to 5, and
a positioning arm (230, 330) according to any one of the claims 6 to 13.

## Patentansprüche

1. Adaptervorrichtung, die dazu eingerichtet ist, mit einem Körperimplantat (210) verbunden zu werden, zum lösbaren Herstellen einer räumlichen Ausrichtung zwischen dem Körperimplantat (210) und einer Zielvorrichtung (280), insbesondere zwischen einem Knochenstabilisierungsimplantat (210) und einer Zielvorrichtung (280) zum Verankern des Implantats (210) an einem inneren Teil eines Körpers, wobei die Adaptervorrichtung (221, 321) aufweist:
ein Befestigungselement (225a, 325a), welches derart gestaltet ist, dass ein Eingriffselement (245a, 345a) eines Positionierungsarms (230, 330) in die Adaptervorrichtung (221, 321) eingehakt werden kann, und
ein Verriegelungselement (327), welches derart gestaltet ist, dass ein Schnappmechanismus (350) des Positionierungsarms (230, 330) selbsttätig in das Verriegelungselement (327) eingreift, wenn der Positionierungsarm (230, 330), wenn er in die Adaptervorrichtung (221, 321) eingehakt wird, bezüglich der Adaptervorrichtung (221, 321) zu einer finalen Winkelstellung hin geschwenkt wird,
**dadurch gekennzeichnet, dass** die Adaptervorrichtung ein weiteres Verriegelungselement (327) umfasst, welches ebenfalls derart gestaltet ist, dass ein Schnappmechanismus (350) des Positionierungsarms (230, 330) selbsttätig in das Verriegelungselement (327) eingreift, wenn der Positionierungsarm (230, 330), wenn er in die Adaptervorrichtung (221, 321) eingehakt wird, bezüglich der Adaptervorrichtung (221, 321) zu einer finalen Winkelstellung hin geschwenkt wird.

2. Adaptervorrichtung nach Anspruch 1, wobei das Befestigungselement (225a, 325a) ein Vorsprung ist, der von einem Körper (326) der Adaptervorrichtung (221, 321) vorspringt.

3. Adaptervorrichtung nach einem der Ansprüche 1 bis 2, wobei jedes der Verriegelungselemente (327) eine Kante umfasst.

4. Adaptervorrichtung nach einem der Ansprüche 1 bis 3, wobei die Adaptervorrichtung (221, 321) mit dem Implantat (210) integral ausgebildet ist.

5. Adaptervorrichtung nach einem der Ansprüche 1 bis 3, wobei die Adaptervorrichtung (221, 321) mit einem internen Fixateur (210), insbesondere mit einer Platte (210), welche für eine Knochenstabilisierung verwendet wird, integral ausgebildet ist.

6. Positionierungsarm zum lösbaren Herstellen einer räumlichen Ausrichtung zwischen einem Körperimplantat (210) und einer Zielvorrichtung (280), insbesondere zwischen einem Knochenstabilisierungsimplantat (210) und einer Zielvorrichtung (280) zum Verankern des Implantats (210) an einem inneren Teil eines Körpers, wobei der Positionierungsarm (230, 330) aufweist:
ein Eingriffselement (245a, 345a), welches dazu ausgebildet ist, an ein Befestigungselement (225a, 325a) einer Adaptervorrichtung (221, 321) gekoppelt zu werden, die dazu eingerichtet ist, mit dem Körperimplantat (210) verbunden zu werden, derart, dass der Positionierungsarm (230, 330) in die Adaptervorrichtung (221, 321) eingehakt ist, und
einen Schnappmechanismus (350), welcher dazu ausgebildet ist, selbsttätig mit einem Verriegelungselement (327) der Adaptervorrichtung (221, 321) in Eingriff zu kommen, wenn der Positionierungsarm (230, 330), wenn er in die Adaptervorrichtung (221, 321) eingehakt wird, bezüglich der Adaptervorrichtung (221, 321) zu einer finalen Winkelstellung hin geschwenkt wird,
**dadurch gekennzeichnet, dass** der Positionierungsarm (230, 330) einen weiteren Schnappmechanismus (350) umfasst, welcher ebenfalls dazu ausgebildet ist, selbsttätig mit einem weiteren Verriegelungselement (327) der Adaptervorrichtung (221, 321) in Eingriff zu kommen, wenn der Positionierungsarm (230, 330), wenn er in die Adaptervorrichtung (221, 321) eingehakt wird, bezüglich der Adaptervorrichtung (221, 321) zu einer finalen Winkelstellung hin geschwenkt wird.

7. Positionierungsarm nach Anspruch 6, wobei das Eingriffselement (245a, 345a) eine Kerbe ist.

8. Positionierungsarm nach einem der Ansprüche 6 bis 7, wobei jeder der Schnappmechanismen (350) ein hakenartiges Element (351) aufweist, welches dazu eingerichtet ist, in eines der Verriegelungselemente (327) einzugreifen.

9. Positionierungsarm nach einem der Ansprüche 6 bis 8, welcher ferner aufweist:
einen Handgriff (290, 390) zum Erleichtern einer manuellen Schwenkung des Positionierungsarms (230, 330) bezüglich der Adaptervorrichtung (221, 321).

10. Positionierungsarm nach einem der Ansprüche 6 bis 9, welcher ferner aufweist:
eine Verrieglungsvorrichtung (360), die dazu eingerichtet ist, einen der Schnappmechanismen (350) zu verriegeln.

11. Positionierungsarm nach Anspruch 10, wobei die Verrieglungsvorrichtung (360) einen drehbaren Hebel (295) aufweist.

12. Positionierungsarm nach einem der Ansprüche 6 bis 11, wobei der Positionierungsarm (230, 330) mit der Zielvorrichtung (280) integral ausgebildet ist.

13. Positionierungsarm nach einem der Ansprüche 6 bis 11, wobei der Positionierungsarm (230, 330) dazu eingerichtet ist, lösbar mit der Zielvorrichtung (280) verbunden zu werden.

14. System zum lösbaren Herstellen einer räumlichen Ausrichtung zwischen einem Körperimplantat (210) und einer Zielvorrichtung (280), insbesondere zwischen einem Knochenstabilisierungsimplantat (210) und einer Zielvorrichtung (280) zum Verankern des Implantats (210) an einem inneren Teil eines Körpers, wobei das System aufweist:
eine Adaptervorrichtung (221, 321) nach einem der Ansprüche 1 bis 5, und
einen Positionierungsarm (230, 330) nach einem der Ansprüche 6 bis 13.

## Revendications

1. Dispositif adaptateur adapté pour être relié à un implant corporel (210) pour établir, de manière amovible, une orientation spatiale entre ledit implant corporel (210) et un dispositif de pointage (280), en particulier entre un implant de stabilisation osseuse (210) et un dispositif de pointage (280) pour ancrer l'implant (210) à une partie interne d'un corps, le dispositif adaptateur (221, 321) comportant :
un élément de fixation (225a, 325a), qui est conçu de telle sorte qu'un élément de prise (245a, 345a) d'un bras de positionnement (230, 330) peut être accroché dans le dispositif adaptateur (221, 321), et
un élément de blocage mutuel (327), qui est conçu de telle sorte qu'un mécanisme d'encliquetage (350) du bras de positionnement (230, 330) vient automatiquement en prise dans l'élément de blocage mutuel (327) lorsque le bras de positionnement (230, 330), une fois accroché dans le dispositif adaptateur (221, 321) est mis en rotation par rapport au dispositif adaptateur (221, 321) vers une position angulaire finale,
**caractérisé en ce que** ledit dispositif adaptateur comporte un élément de blocage mutuel supplémentaire (327), qui est également conçu de telle sorte qu'un mécanisme d'encliquetage (350) du bras de positionnement (230, 330) vient automatiquement en prise dans l'élément de blocage mutuel (327) lorsque le bras de positionnement (230, 330), une fois accroché dans le dispositif adaptateur (221, 321), est mis en rotation par rapport au dispositif adaptateur (221, 321) vers une position angulaire finale.

2. Dispositif adaptateur selon la revendication 2, dans lequel
l'élément de fixation (225a, 325a) est une protubérance en saillie à partir d'un corps (326) du dispositif adaptateur (221, 321).

3. Dispositif adaptateur selon l'une quelconque des revendications 1 à 2, dans lequel
chacun des éléments de blocage mutuel (327) comporte un bord.

4. Dispositif adaptateur selon l'une quelconque des revendications 1 à 3, dans lequel
le dispositif adaptateur (221, 321) est formé d'un seul tenant avec l'implant (210).

5. Dispositif adaptateur selon l'une quelconque des revendications 1 à 3, dans lequel
le dispositif adaptateur (221, 321) est formé d'un seul tenant avec un fixateur interne (210), en particulier avec une plaque (210) qui est utilisée pour une stabilisation osseuse.

6. Bras de positionnement pour établir, de manière amovible, une orientation spatiale entre un implant corporel (210) et un dispositif de pointage (280), en particulier entre un implant de stabilisation osseuse (210) et un dispositif de pointage (280) pour ancrer l'implant (210) à une partie interne d'un corps, le bras de positionnement (230, 330) comportant :
un élément de prise (245a, 345a), qui est conçu pour être couplé à un élément de fixation (225a, 325a) d'un dispositif adaptateur (221, 321) adapté pour être relié audit implant corporel (210) de telle sorte que le bras de positionnement (230, 330) est accroché dans le dispositif adaptateur (221, 321), et
un mécanisme d'encliquetage (350), qui est conçu pour venir automatiquement en prise dans un élément de blocage mutuel (327) du dispositif adaptateur (221, 321), lorsque le bras de positionnement (230, 330), une fois accroché dans le dispositif adaptateur (221, 321), est mis en rotation par rapport au dispositif adaptateur (221, 321) vers une position angulaire finale,
**caractérisé en ce que** ledit bras de positionnement (230, 330) comporte un mécanisme d'encliquetage supplémentaire (350), qui est également conçu pour venir automatiquement en prise dans un élément de blocage mutuel supplémentaire (327) du dispositif adaptateur (221, 321), lorsque le bras de positionnement (230, 330), une fois accroché dans le dispositif adaptateur (221, 321), est mis en rotation par rapport au dispositif adaptateur (221, 321) vers une position angulaire finale.

7. Bras de positionnement selon la revendication 6, dans lequel
l'élément de prise (245a, 345a) est une encoche.

8. Bras de positionnement selon l'une quelconque des revendications 6 à 7, dans lequel
chacun des mécanismes d'encliquetage (350) comporte un élément de type crochet (351), qui est adapté pour venir en prise dans l'un des éléments de blocage mutuel (327).

9. Bras de positionnement selon l'une quelconque des revendications 6 à 8, comportant en outre
une poignée (290, 390) pour faciliter une rotation manuelle du bras de positionnement (230, 330) par rapport au dispositif adaptateur (221, 321).

10. Bras de positionnement selon l'une quelconque des revendications 6 à 9, comportant également
un dispositif de blocage (360), adapté pour bloquer l'un des mécanismes d'encliquetage (350).

11. Bras de positionnement selon la revendication 10, dans lequel
le dispositif de blocage (360) comporte un levier rotatif (295).

12. Bras de positionnement selon l'une quelconque des revendications 6 à 11, dans lequel
le bras de positionnement (230, 330) est formé d'un seul tenant avec le dispositif de pointage (280).

13. Bras de positionnement selon l'une quelconque des revendications 6 à 11, dans lequel
le bras de positionnement (230, 330) est adapté pour être relié au dispositif de pointage (280) de manière amovible.

14. Système pour établir, de manière amovible, une orientation spatiale entre un implant corporel (210) et un dispositif de pointage (280), en particulier entre un implant de stabilisation osseuse (210) et un dispositif de pointage (280) pour ancrer l'implant (210) à une partie interne d'un corps, le système comportant :
un dispositif adaptateur (221, 321) selon l'une quelconque des revendications 1 à 5, et
un bras de positionnement (230,330) selon l'une quelconque des revendications 6 à 13.
